# EUROPEAN PATENT SPECIFICATION

(11) **EP 0 460 917 B1**
(45) Date of publication and mention of the grant of the patent: **02.01.1997**
(21) Application number: 91305044.9
(22) Date of filing: 04.06.1991
(51) Int. Cl.: C07C 51/44, C07C 57/03, C07C 67/54, C07C 69/587

(54) **Method of producing eicosapentaenoic acid or an ester derivative thereof**
Verfahren zur Herstellung von Eicosapentaensäure oder von einem Esterderivat davon
Procédé de fabrication de l'acide eicosapentaénoique ou un de ses dérivés esters

(30) Priority: 04.06.1990 JP 145617/90; 04.06.1990 JP 145618/90
(43) Date of publication of application: 11.12.1991
(73) Proprietor: NIPPON SUISAN KAISHA, LTD., Tokyo 100 (JP); KANSAI CHEMICAL ENGINEERING CO. LTD, Amagasaki-shi Hyogo 660 (JP)
(72) Inventor: Hata, Kazuhiko, Kanagawa (JP); Noda, Hideo, Iiyogo (JP); Makuta, Masahiro, Tokyo (JP)
(74) Representative: Holmes, Michael John

(56) References cited:
- WO-A-89/11521
- DE-A- 2 220 922
- FR-A- 2 509 622
- US-A- 3 891 511
- US-A- 4 377 526
- DATABASE WPIL Week 8242, Derwent Publications Ltd., London, GB; AN 82-89665E
- PATENT ABSTRACTS OF JAPAN vol. 7, no. 77 (C-159)(1222) 30 March 1983
- CHEMICAL ABSTRACTS, vol. 114, no. 12, 25 March 1991, Columbus, Ohio, US; abstract no. 104748p, page 135 ;column 2 ;
- CHEMICAL ABSTRACTS, vol. 115, no. 18, 4 November 1991, Columbus, Ohio, US; abstract no. 185702q, NODA YASUO ET AL. 'Purification of eicosapentaenoic ethyl ester by distillation under high vacuum.' page 130 ;column 2 ;
- Derwent abstract AN=83-775755

## Description

The present invention relates to a method for producing high-concentration eicosapentaenoic acid or the ester derivative thereof, more specifically to a new method of producing, at high efficiency, a high-concentration form of eicosapentaenoic acid (EPA) or the ester derivative thereof effective as a prescription medicine for the prevention of thrombosis and the medical treatment and prevention of thrombus-induced diseases.

Eicosapentaenoic acid (EPA) or the ester, amide or other derivatives thereof have been conventionally known as effective prescription medicines for the prevention of thrombosis and for the medical treatment of thrombus-induced diseases.

It is known that eicosapentaenoic acid (EPA) or the derivatives thereof are contained per se or in the form of the glyceride and other derivatives thereof, in natural fats and oils, particularly in fats and oils of mackerel, sardine, cod and other marine products. Studies have bean made concerning the methods for removing eicosapentaenoic acid from these fish oils. Thus, for example, US-A-4377526 describes the purification of EPA by subjecting a mixture of fatty acids to urea treatment and to distillation using two fractionating columns.

However an overwhelming quantity of fatty acids wherein those with a carbon number of less than 19 and more than 21, other than eicosapentaenoic acid (an unsaturated fatty acid with a carbon number of 20) are mixed in the natural fats and oils in fish oils. This makes it exceedingly difficult to efficiently and selectively remove eicosapentaenoic acid alone as a high-concentration (high purity) product.

For example, a method has been proposed in which fatty acid mixtures from natural fats and oils are esterified, and put to precision fraction under reduced pressures and the resultant fraction is purified using an urea addition method to provide eicosapentaenoic acid from natural fats and oils (Japanese Patent Provisional Publication No. 149400/82).

By this method by which fatty acid mixtures from natural fats and oils are subjected to precision fraction under reduced pressures of 10mmHg (1333 Pa), preferably of 0.1 to 0.01mmHg (13.3 to 1.33 Pa) with a single rectifying column filled with rings and the resulting products are rectified by the urea addition method, eicosapentaenoic acid with a concentration of some 80% can be obtained. By this method, however, a mere 30% of eicosapentaenoic acid ester exists in the C₂₀ fraction obtained by the rectification, and also it is necessary to adopt complicated troublesome treatment processes, such as the treatment of urea adduct and the subsequent distillation under reduced pressures. Even with these processes, it is exceedingly difficult to improve the concentration of eicosapentaenoic acid ester to 85% or higher. By the foregoing method which was publicly known, large quantities and a multiplicity of treatments with urea become practically required, placing great limitations on the reduction of manufacturing cost as well an the improvement of production efficiency. There has been a serious limitation to putting the method into practical application.

Almost concurrently with this publicly known method, a method was proposed by the applicant of the present invention wherein two distillation columns are employed to subject fatty acid mixtures from natural fats and oils to continuous distillation to provide approximately 50% eicosapentaenoic acid as C₂₀ fraction. The product is then subjected to an urea addition treatment and purification through column chromatography (Japanese Patent Provisional Publication No. 8037/1983. This improved method has substantially enhanced the efficiency of distillation purification process and the whole process, but eicosapentaenoic acid and the ester derivative thereof with a concentration (or purity) as high as 80% or more cannot be produced without the subsequent urea addition treatments. Even with the urea addition process, the method still remains unsuccessful in producing eicosapentaenoic acid and the ester derivative thereof with a concentration as high as 85% or higher. For this reason, there has been a limit to the rationalization of the production process and the improvement of production efficiency.

FR-A-2509622 describes the use of multi-column fractional distillation in the production of butan-1,4-diol, and US-A-3891511 describes the use of multi-column fractionation in butadiene monomer purification. SU-0977447 describes the rectification of inter-alia fatty acids and alcohols.

In order to use eicosapentaenoic acid and the ester derivative thereof, effective as a medical prescription medicine in studies for clinical purposes or for a wider range of diseases, it is strongly desired to produce these compounds with a concentration of 80% or more, particularly 85% or more in large quantities and at high efficiency. Yet, under the aforestated circumstances, such demand has to date been unable to be met.

The present invention has been made considering the aforestated circumstances, and it is designed to provide a method which makes it possible to overcome the shortcomings of the conventional production and purification methods and provide eicosapentaenoic acid and the ester derivative thereof with a concentration higher than 80%, preferably higher than 85%, in a convenient fashion, at high efficiency and low costs.

In order to overcome the foregoing problems, the present invention provides a method for producing eicosapentaenoic acid or an ester derivative thereof at a purity of at least 80%, said method comprising subjecting a mixture of fatty acids and/or esters thereof obtained from natural fats and oils, and including eicosapentaenoic acid and/or an ester thereof, to continuous distillation in a distillation system comprising at least 3 distillation columns, said distillation occurring under a pressure of ≦10 Torr (approx. 1333 Pa) and a temperature at the bottom of each column of ≦210°C, at least one of said columns being a separate rectifying column for an initial fraction of fatty acids having carbon numbers less than 20 obtained from a first distillation column, the liquid at the bottom of said rectifying column being refluxed to said first distillation column.

In the method according to the present invention, it is preferred to send the condensate of the top fraction of a pre-stage distillation column to said rectifying column for the initial fraction, and to establish a rectifying column for the main fraction which contains eicosapentaenoic acid and the ester derivative thereof and that for the residue independently for continuous distillation.

The present invention also provides a method of producing eicosapentaenoic acid and the ester derivative thereof by subjecting fatty acids or the ester mixtures thereof obtained from the natural fats and oils including eicosapentaenoic acid and the ester derivative thereof to continuous distillation under a reduced pressure of 10 Torr (1333 Pa) or below and a bottom temperature of 210°C or less, by means of refluxing, to a pre-stage distillation column bottom liquid at a rectifying column in a distillation column system comprised of 3 or more distillation columns, with the rectifying column for the initial fraction of low-carbon number fatty acids separated, bringing the main fraction containing the resultant eicosapentaenoic acid and the ester derivative thereof into contact with a solution of urea in methanol to give urea adduct, putting the same to a drainage treatment with non-polar solvent and removing the non-polar solvent to provide eicosapentaenoic acid and the ester derivative thereof with a concentration as high as 85% or more.

The invention will be described with reference to the accompanying drawings, in which:
FIG. 1 is a system construction drawing illustrating one embodiment of a method of producing eicosapentaenoic acid and the ester derivative thereof through continuous distillation.
FIG. 2 is a system construction drawing illustrating urea-addition treatment processes following the continuous distillation.
FIG. 3 is a system construction drawing illustrating a purification process following the urea addition.
FIG. 4 is a system construction drawing depicting the conventional two-column distillation method.

Eicosapentaenoic acids and other long-chain high-degree unsaturated fatty acids contain so many double bonds in the molecules thereof that they are apt to be subjected to degradation, polymerisation and other thermal denaturation due to heating in distillation, rendering condensation through distillation exceedingly difficult.

On the other hand, the natural fats and oils containing eicosapentaenoic acids contain various kinds of fatty acids other than eicosapentaenoic acids, which have boiling points closer to each other, and hence they cannot be separated unless the distillation column is made fairly high and the amount of reflux is increased. However, this causes thermal denaturation due to rises in the bottom pressure and the resultant temperature rise, making distillation purification exceedingly difficult.

For these reasons, by the conventional methods, distillation condensation is controlled to a low level and it is necessary to purify the fatty acids to a high degree through urea addition and other processes at subsequent stages. Inevitably, the load on the processes at the subsequent stages becomes larger.

The method according to the present invention provides eicosapentaenoic acid or the ester derivative thereof with a purity as high as 80% or preferably 85% or more without causing any problems, in a simple operation requiring only distillation purification, and at high efficiency. It also becomes possible to purify the resultant eicosapentaenoic acid or the ester derivative thereof to a product with a concentration as high as 90% at extremely high efficiency by the subsequent urea addition processes.

As the fatty acid mixtures applicable to the method according to the present invention, any substances obtainable from the natural fats and oils which contain quantities of eicosapentaenoic acid or the glyceride derivative thereof can be used. For instance, fatty acid mixtures from sardine, mackerel, herring, saury and other fish, and Antarctic euphausian, and other animal marine plankton.

These fatty acid mixtures are esterified for continuous distillation as desired.

As the continuous distillation method according to the present invention, filling spring, column plate and various other systems can be employed, and more preferably, a mesh plate can be used with a theoretical number of plates more than 5.

Continuous distillation using 3 or more distillation columns according to the present invention is accomplished under a reduced pressure of approximately 0.1 Torr (13.3 Pa) and at 210°C or below, or more preferably, at a bottom temperature of 195°C or less.

The construction of this three-or-more column distillation columns is such that, one of the columns is made independent as a rectifying column for the collection of initial fraction. For instance, in the case of such three-column construction, the columns are divided as follows:
(I) First distillation column;
(II) Second distillation column (rectifying column for initial fraction); and
(III) Third distillation column (rectifying column for main fraction and residual fraction).

In the case of four-column construction, the columns are divided as follows:
(I) First distillation column;
(II) Second distillation column (rectifying column for initial fraction);
(III) Third distillation column (rectifying column for residual fraction); and
(IV) Fourth distillation column (rectifying column for main fraction).

Furthermore, in the case of three-column construction, the columns can be divided as follows:
(I) First distillation column (rectifying column for initial fraction);
(II) Second distillation column (rectifying column for residual fraction); and
(III) Third distillation column (rectifying column for main fraction).
Needless to say, the construction of the rectifying columns can be further divided.

In any of the above cases, in the method according to the present invention, it is essential that the bottom liquid of the rectifying column for the initial fraction be returned as reflux liquid to the first distillation column as in the above example. It is also preferable to condense the top fraction of the first distillation column, then send it as a condensate to the rectifying column for the initial fraction.

The main fraction of C₂₀ obtainable through continuous distillation, ie., the fraction containing eicosapentaenoic acid or the ester derivative is then subjected to urea treatment to provide the urea adduct. Then, the urea adduct is dissolved in methanol, ethanol and other highly soluble solvents to be used as an urea adduct solution. Normally, the concentration of urea adduct is 5 to 20%.

Mixing the main fraction with this urea adduct solution is accomplished at a ratio of 0.5 to 10 part to 1 part by weight of the main fraction. The mixture is subjected to forced cooling to room temperature or below, more preferably to 15°C or less. Such treatment permits C₂₀ fatty acids having a lower degree of unsaturation, for example, 1 to 4 unsaturated bonds, to be separated as a compound material with urea.

Then, in the method according to the present invention, non-polar solvents, for instance, hexane and isooctane are added to the reaction mixture. The urea adduct and remaining urea are caused to shift to the methanol layer, and the fatty acids to the hexane layer for extraction and separation.

Then, in order to remove colouring matter, oxides and other impurities as needed, the resulting substances are subjected to adsorption treatment via an adsorption column.

As the adsorption column, silica gel, activated clay, alumina and activated carbon can be used, but silica gel is the most preferred. Thereafter, the aforementioned solvents are removed through distillation.

While referring to the drawings attached, more detailed descriptions will be given to the method according to the present invention.

### (Continuous distillation)

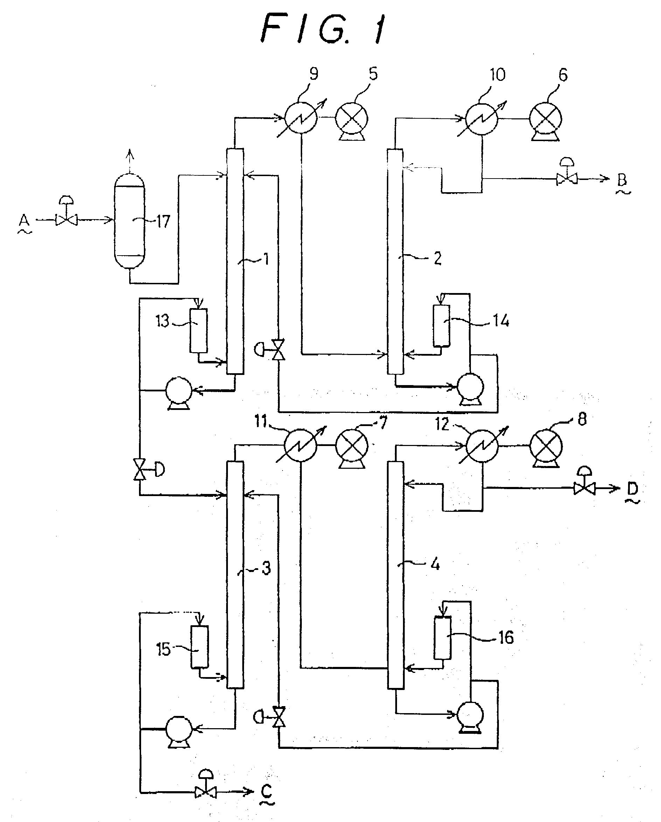

FIG. 1 shows an example in which four distillation columns are used.

For instance, as indicated in FIG. 1, the fatty acid mixture (A) is subjected to continuous distillation using four distillation columns (1), (2), (3) and (4).

At each of the distillation columns (1), (2), (3) and (4), vacuum systems (5), (6), (7) and (8), the condensation systems (9), (10), (11) and (12) and reboilers (13), (14), (15) and (16) are also provided.

These distillation columns (1), (2), (3) and (4) are strictly controlled to a reduced pressure of 10 Torr (1333 Pa) or below, and at a bottom temperature of 210°C or lower. The degree of vacuum is closely associated with temperature, and it is preferred but not essential that the vacuum systems (5), (6), (7) and (8) are made independent from one another. It is permissible to construct these vacuum systems arbitrarily in accordance with the capability and control systems of the vacuum pumps.

Under the aforestated construction, raw material (A) is introduced into the first distillation column (1), for instance, in the vicinity of the top thereof, the top fraction is condensed at the condensate system (9) and is introduced in liquid form into a rectifying column as the second distillation column (2), for example, at the bottom thereof. Such introduction of the top fraction in liquid form is one of the important factors in the method according to the present invention.

In the second distillation column (2), the initial fraction (B) consisting of fatty acids having lower carbon number (< C₁₉) as the top fraction thereof is collected. Some of the bottom liquid is refluxed to the vicinity of the top of the first distillation column (1). This is also a very important factor to the method of the present invention. The bottom condensate of the first distillation column (1) is heated with a reboiler (13) and returned to the bottom thereof, and at the same time, introduced in liquid form to the vicinity of the top of the third distillation column (3).

The top fraction of the third distillation column (3) is supplied to the bottom of the fourth distillation column (4) as condensate through a condensation system (11). The bottom condensate is heated with a reboiler (13) and returned to the bottom thereof, and concurrently the remaining fraction (C) consisting primarily of C₂₁ or greater fatty acids with longer chains than eicosapentaenoic acid or the ester derivative.

At the fourth distillation column (4) into which condensate has been introduced from the top of the third distillation column (3), the fraction from the top is condensed at the condensation system (12), some of them being refluxed to the vicinity of the top and the main fraction (D) consisting primarily of eicosapentaenoic acid or the ester derivative. On the other hand, the bottom condensate is heated with a reboiler (16) and returned to the bottom, and concurrently some of them is refluxed to the vicinity of the third distillation column (3).

Raw material (A) is treated at a flash tank (17) maintained at a reduced pressure prior to the introduction thereof into the first distillation column (1), removing air, water content and other impurities. It is advantageous to use falling film evaporation type reboilers as reboilers (13), (14), (15) and (16). This can effectively prevent thermal degradation.

### (Continuous distillation and urea addition treatment)

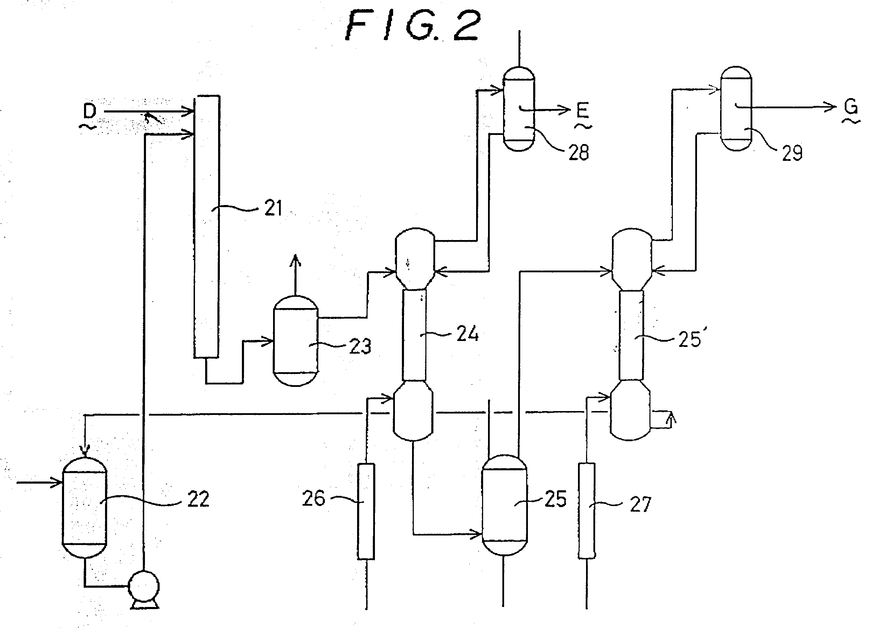

FIG. 2 illustrates the treatment process of the formation of the urea adduct. The main fraction (D) containing eicosapentaenoic acid or the ester derivative thereof with a concentration of 80% or higher was obtained by the foregoing continuous distillation.

As shown in FIG. 2, the main fraction (D) is sent to a column in contact with a solution of urea, and, for instance, a solution of urea in methanol is guided from a tank (22) to be brought into contact with the column. At this time, the solution of urea in methanol is introduced at a temperature of 35 to 45°C, and is subjected to forced cooling at the column in contact therewith so that it is at or below room temperature.

Then, the treatment liquid is fed through a tank (23) to a product extraction column (24) using non-polar solvent, eg., n-hexane. The product solvent layer (E) which separates the solution of urea in methanol and the urea adduct is sent to the next process. The solution of urea in methanol and the urea adduct are fed to a tank (25), and after being subjected to thermal decomposition, they are put to another cycle of extraction treatment at a residue extracting column (25') to separate a residue solvent layer (G). The non-polar solvent is either cooled or heated at a cooling column (26) or a heating column (27) and fed to the extracting columns (24,25'). The solvent layers (E,G) are taken out of decanters (28,29).

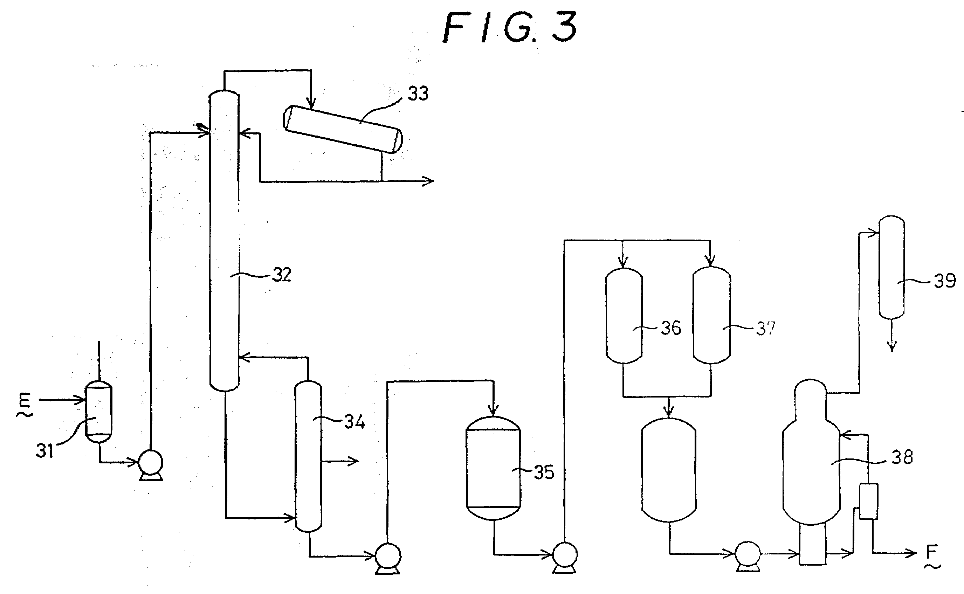

Then, the product solvent layer (E) is fed to a methanol rectifying column (32) through a tank (31) as indicated in FIG. 3. By the operation at the methanol rectifying column (32) in which a rectifying condenser (33) and a methanol evaporator (34) are provided, a product extracting solvent layer which is subjected to a treatment of removing methanol is obtained, and filtering the same removes the remaining urea.

Then, this substance is led to adsorption columns (36,37) through a tank (35), removing colouring matter, oxides and other impurities. Next, solvent, eg., n-hexane, is removed using an evaporator (38).

An n-hexane condenser (39) is provided for this evaporator.

In this way, a product (F) can be obtained. The final concentration of the product (F) is increased to 85% or higher.

Specific examples of production by the production method of the present invention will be given using the systems as indicated in FIGs. 1 to 3.

### (Production example 1)

The ethylester in a fatty acid mixture (60% for C₁₉, 23% for C₂₀ and 17% for C₂₁) obtained from fish oil was treated in a flask tank (17) which is kept at a vacuum of 1 Torr (133 Pa), then supplied to the first distillation column (1) with a column diameter of 300mm and a height of approx. 7m and maintained at a vacuum of 0.1 Torr (13.3 Pa) at a rate of 15 to 20 l/hr.

In this first distillation column (1), the bottom temperature was controlled at 194 to 195°C, and the top temperature at 124 to 125°C. Inside it, a 4mm-mesh plate was provided, with the theoretical number of plates set at 4. Since fatty acid ester mixtures greater than C₂₀ are collected at the bottom of this first distillation column (1), it becomes difficult to control the degree of vacuum and temperature at the bottom thereof. Accordingly, the quantity of packing into the first distillation column was less than that into the second distillation column (2).

The top condensate of the first distillation column (1) was introduced into the bottom of the second distillation column (2). The bottom temperature of this second column was set at 184 to 185°C, while the top temperature was set at 100 to 111°C, with the column operated under a reduced pressure of 0.1 Torr (13.3 Pa). The theoretical number of column plates was set at 6. The top fraction was refluxed at a reflux ratio of 1:2, some of them being collected as the initial fraction (B).

The composition of the initial fraction was, as indicated in Table 1, 99% for C₁₉ or lower fatty acids, 1% for C₂₀ eicosapentaenoic acid ester and others and 0% for C₂₁ or greater fatty acids.

The second distillation column (2) was controlled so that the bottom condensate thereof was constant as level, and said condensate was returned to the vicinity of the top of the first distillation column (1). In other words, the bottom condensate was returned as reflux liquid to the first distillation column (1).

The bottom liquid of the first distillation column (1) was fed to the vicinity of the top of the third distillation column (3). The pressure of the third distillation column (3) was a reduced pressure of 0.1 Torr (13.3 Pa), while the bottom temperature was 194 to 195°C and the top temperature at 124 to 125°C. The theoretical number of column plates was 4.

As the bottom liquid of the third distillation column (3), the residual fraction (C) was collected. The residual fraction was, as indicated in Table 1, composed of 0.1% for C₁₈ or lower fatty acids, 20% for C₂₀ eicosapentaenoic acid ester and others and 79.9% for C₂₁ or greater fatty acids.

The top fraction of this third distillation column (3) was supplied to the fourth distillation column (4) as condensate. The fourth distillation column (4) with a theoretical number of column plates of 6 was operated under a reduced pressure of 0.1 Torr (13.3 Pa), at a bottom temperature of 194 to 195°C and a top temperature of 110 to 111°C.

The bottom liquid was returned as reflux liquid to the top of the third distillation column (3). The level of the bottom liquid of the fourth distillation column was kept constant.

The top condensate was refluxed at a reflux ratio of 1 to 2, and at the same time, the main fraction (D) was collected.

The main fraction was, as indicated in Table 1, composed of 0.1% for C₁₉ or lower fatty acids, 0% for C₂₁ or greater fatty acids and 99.9% for C₂₀ eicosapentaenoic acid ester and others.

The concentration of eicosapentaenoic acid ethylester in the C₂₀ fraction was 88%.

### (Comparative example)

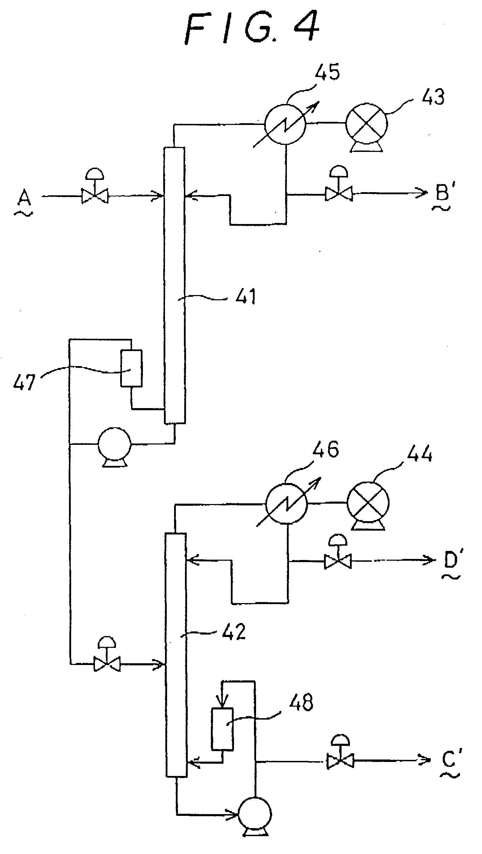

For comparison, attempts were made to perform continuous distillation under reduced pressure using a two-column distillation column system (41,42) (theoretical number of column plates being 10 as shown in FIG. 4.

Again in this case, each of the distillation columns (41,42) was provided with independent vacuum systems (43,44) and condensation systems (45,46), and also with a reboiler (48).

The system was so designed that the initial fraction (B') was collected from the top of the first distillation column (41), the main fraction (D') from the top of the second distillation column (42) and the residual fraction (C') from the bottom thereof. Each of the distillation columns (41,42) was under a reduced pressure of 0.1 Torr (13.3 Pa). Inspite of the attempts to control the bottom temperature of the first distillation column (41) to 195°C or lower, it was difficult to control temperatures; the bottom temperature could rise to 210°C or higher.

The compositions of the initial fraction, main fraction and residual fraction were as indicated in Table 2. The efficiency of separation/purification of C₂₀ fraction by this example proved to be by far inferior to the method of the present invention, making it exceedingly difficult to control the distillation operation. Only 76% of the eicosapentaenoic acid ethylester of the main fraction which was collected as C₂₀ fraction was collected. Even though the bottom temperature of the first distillation column (41) was controlled to 195°C or lower, as is evident from Table 2, fatty acids with lower carbon number, particularly C₁₈ fatty acids, were inevitably included, rendering the resultant product completely unsatisfactory.

### (Production example 2)

The main fraction (D) which contains 99.9% C₂₀ ethylester and 88% eicosapentaenoic acid ethylester, both obtained from Example 1, was brought into contact with a 15% solution of urea in methanol in a contact column (21). The temperature of this solution was 42°C, and it was subjected to forced cooling down to 10°C in the contact column (21).

Using the n-hexane cooled to 10°C, the product was subjected to drainage treatment in an extraction column (24).

The product solvent layer (E) obtained was freed from methanol in a methanol-removing column (32), and further urea was filtered. The product solvent layer (E) contained the following components, but the methanol and urea were completely removed.

| | |
|---|---|
| Fatty acid ester | 12 to 13% |
| Methanol | 5% |
| Hexane | 82 to 83% |
| Trace amount of urea | |

The treatment liquid was then guided to silica gel adsorption columns (36,37), colouring matter, oxides and other impurities were removed, and hexane was removed in an evaporator (38), providing eicosapentaenoic acid ethylester product (F). The concentration thereof was 93%.

As has been described in detail, the method of the present invention can provide eicosapentaenoic acid or the ester derivative thereof having a concentration (purity) of 85% or higher, and further 90% or higher. It can also produce the product at high efficiency.

**Table 1**

| Composition | Raw Material (A) | Initial Fraction (B) | Residual Fraction (C) | Main Fraction (product) (D) |
|---|---|---|---|---|
| <C₁₉ | 60 | 99 | 0.1 | 0.1 |
| C₂₀ | 23 | 1 | 20 | 99.9 |
| C₂₁< | 17 | 0 | 79.9 | 0 |

**Table 2**

| Composition | Raw Material (A) | Initial Fraction (B') | Residual Fraction (C') | Main Fraction (product) (D') |
|---|---|---|---|---|
| <C₁₉ | 60 | 80 | 5.1 | 10.1 |
| C₂₀ | 23 | 20 | 20.0 | 89.9 |
| C₂₁< | 17 | 0 | 74.9 | 0 |

## Claims

1. A method for producing eicosapentaenoic acid or an ester derivative thereof at a purity of at least 80%, said method comprising subjecting a mixture of fatty acids and/or esters thereof obtained from natural fats and oils, and including eicosapentaenoic acid and/or an ester thereof, to continuous distillation in a distillation system comprising at least 3 distillation columns, said distillation occurring under a pressure of ≦10 Torr (approx. 1333 Pa) and a temperature at the bottom of each column of ≦210°C, at least one of said columns being a separate rectifying column for an initial fraction of fatty acids having carbon numbers less than 20 obtained from a first distillation column, the liquid at the bottom of said rectifying column being refluxed to said first distillation column.

2. A method as claimed in claim 1, wherein the condensate of the top fraction is conveyed from said first distillation column to said rectifying column as said initial fraction.

3. A method as claimed in claim 1 or claim 2 wherein said liquid at the bottom of the rectifying column is refluxed to the vicinity of the top of a pre-stage distillation column.

4. A method as claimed in any one of the preceding claims, wherein a rectifying column for the main fraction which contains eicosapentaenoic acid and/or an ester derivative thereof as main constituent, and a rectifying column for the residual fraction of high-carbon number fatty acids are independent from each other for continuous distillation.

5. A method as claimed in any one of the preceding claims wherein each of the distillation columns has its own independent vacuum systems and condensation systems.

6. A method as claimed in any one of the preceding claims, further comprising bringing into contact with a solution of urea the main fraction containing the thus-obtained eicosapentaenoic acid and/or ester derivative thereof to give a urea adduct, by subjecting the product to extraction treatment using non-polar solvent, then removing the solvent through distillation.

7. A method as claimed in claim 6 wherein the eicosapentaenoic acid or ester derivative thereof is produced at a purity of at least 85%.

8. A method as claimed in claim 6 or claim 7, wherein a solution of urea in methanol and the main fraction are brought into contact with each other at temperatures lower than room temperature.

9. A method as claimed in any one of claims 6 to 8, wherein said non-polar solvent is hexane.

## Patentansprüche

1. Verfahren zur Erzeugung von Eicosapentaensäure oder eines Esterderivats davon in einer Reinheit von mindestens 80 %, wobei dieses Verfahren umfaßt das Unterwerfen eines Gemisches von Fettsäuren und/oder Estern davon, erhalten aus natürlichen Fetten und Ölen, und einschließend Eicosapentaensäure und/oder einen Ester davon, der kontinuierlichen Destillation in einem Destillationssystem, das wenigstens drei Destillationskolonnen umfaßt, wobei diese Destillation unter einem Druck von ≦ 10 Torr (etwa 1333 Pa) und einer Temperatur am Boden jeder Kolonne von ≦ 210 °C durchgeführt wird, wobei mindestens eine dieser Kolonnen eine separate Rektifizierkolonne für eine Eingangsfraktion von Fettsäuren mit Kohlenstoffzahlen weniger als 20 ist, erhalten aus einer ersten Destillationskolonne, wobei die Flüssigkeit am Boden dieser Rektifizierkolonne zu dieser ersten Destillationskolonne unter Rückfluß rückgeführt wird.

2. Verfahren gemäß Anspruch 1, wobei das Kondensat der Kopffraktion aus dieser ersten Destillationskolonne zu dieser Rektifizierkolonne als die genannte Eingangsfraktion geleitet wird.

3. Verfahren gemäß Anspruch 1 oder Anspruch 2, wobei diese Flüssigkeit am Boden der Rektifizierkolonne in die Nähe des Kopfs einer Vorstufendestillationskolonne refluxiert wird.

4. Verfahren gemäß einem der vorhergehenden Ansprüche, wobei eine Rektifizierkolonne für die Hauptfraktion, welche Eicosapentaensäure und/oder ein Esterderivat davon als Hauptbestandteil enthält, und eine Rektifizierkolonne für die restliche Fraktion von Fettsäure mit hoher Kohlenstoffanzahl unabhängig voneinander für die kontinuierliche Destillation sind.

5. Verfahren gemäß einem der vorhergehenden Ansprüche, wobei jede der Destillationskolonnen seine eigenen unabhängigen Vakuumsysteme und Kondensationssysteme hat.

6. Verfahren gemäß einem der vorhergehenden Ansprüche, welches weiterhin das in-Kontakt-bringen der Hauptfraktion, die die so erhaltene Eicosapentaensäure und/oder Esterderivate davon enthält, mit einer Lösung von Harnstoff umfaßt, um ein Harnstoffaddukt zu ergeben, indem das Produkt der Extraktionsbehandlung unter Verwendung eines nichtpolaren Lösungsmittels unterworfen wird, dann Entfernung des Lösungsmittels durch Destillation.

7. Verfahren gemäß Anspruch 6, wobei Eicosapentaensäure oder ein Esterderivat davon in einer Reinheit von mindestens 85 % erzeugt wird.

8. Verfahren gemäß Anspruch 6 oder Anspruch 7, wobei eine Lösung von Harnstoff in Methanol und die Hauptfraktion in Kontakt miteinander gebracht werden bei Temperaturen niedriger als Raumtemperatur.

9. Verfahren gemäß einem der Ansprüche 6 bis 3, worin dieses nichtpolare Lösungsmittel Hexan ist.

## Revendications

1. Procédé de fabrication de l'acide eicosapentaénoique ou d'un de ses dérivés esters, à une pureté d'au moins 80 %, ledit procédé comprenant le fait de soumettre un mélange d'acides gras et/ou de leurs esters, obtenu à partir de graisses et d'huiles naturelles et incluant l'acide eicosapentaénoique et ou l'un de ses esters, à une distillation continue dans un système de distillation comprenant au moins 3 colonnes de distillation, ladite distillation se produisant sous une pression ≦ 10 Torr (approx. 1333 Pa) et à une température au fond de chaque colonne ≦ 210°C, l'une au moins desdites colonnes étant une colonne de rectification séparée pour une fraction initiale d'acides gras ayant des nombres d'atomes de carbone inférieurs à 20, obtenue à partir d'une première colonne de distillation, le liquide au fond de ladite colonne de rectification étant renvoyé comme reflux à ladite première colonne de distillation.

2. Procédé selon la revendication 1, dans lequel le condensat de la fraction de tête est transféré de ladite première colonne de distillation à ladite colonne de rectification en tant que ladite fraction initiale.

3. Procédé selon la revendication 1 ou la revendication 2, dans lequel ledit liquide au fond de la colonne de rectification est envoyé comme reflux à proximité de la tête d'une colonne de distillation préliminaire.

4. Procédé selon l'une quelconque des revendications précédentes, dans lequel une colonne de rectification pour la fraction principale, qui contient de l'acide eicosapentaénoique et/ou l'un de ses dérivés esters comme constituant principal, et une colonne de rectification pour la fraction résiduelle d'acides gras à nombre de carbones élevé, sont indépendantes l'une de l'autre pour la distillation continue.

5. Procédé selon l'une quelconque des revendications précédentes, dans lequel chacune des colonnes de distillation a son propre système de vide et son propre système de condensation indépendants.

6. Procédé selon l'une quelconque des revendications précédentes, comprenant en outre la mise en contact avec une solution d'urée de la fraction principale contenant l'acide eicosapentaénoique et/ou l'un de ses dérivés esters ainsi obtenu, pour donner un produit d'addition de l'urée, par application au produit d'un traitement d'extraction utilisant un solvant non polaire, puis élimination du solvant par distillation.

7. Procédé selon la revendication 6, dans lequel l'acide eicosapentaénoique, ou l'un de ses dérivés esters, est produit à une pureté d'au moins 85 %.

8. Procédé selon la revendication 6 ou la revendication 7, dans lequel une solution d'urée dans du méthanol et la fraction principale sont mises en contact l'une avec l'autre à des températures inférieures à la température ambiante.

9. Procédé selon l'une quelconque des revendications 6 à 8, dans lequel ledit solvant non polaire est l'hexane.
